(19) Europäisches Patentamt
European Patent Office
Office européen des brevets

(11) Veröffentlichungsnummer: **0 133 895**
**B1**

(12) # EUROPÄISCHE PATENTSCHRIFT

(45) Veröffentlichungstag der Patentschrift: **10.10.90**

(21) Anmeldenummer: **84107305.9**

(22) Anmeldetag: **26.06.84**

(51) Int. Cl.$^5$: **G 01 N 33/50,** G 01 N 33/52, C 12 Q 1/00, A 61 K 35/16, A 61 K 35/18

(54) **Erythrozyten-Rückhaltesubstrate.**

(30) Priorität: **02.07.83 DE 3323973**

(43) Veröffentlichungstag der Anmeldung: **13.03.85 Patentblatt 85/11**

(45) Bekanntmachung des Hinweises auf die Patenterteilung: **10.10.90 Patentblatt 90/41**

(84) Benannte Vertragsstaaten: **AT BE CH DE FR GB IT LI LU NL SE**

(56) Entgegenhaltungen:
**EP-A-0 033 707**
**EP-A-0 045 476**
**DE-A-2 038 722**
**GB-A-1 037 155**
**US-A-3 552 925**
**US-A-3 552 928**

**Handbook for Chemistry and Physics 62nd Edition, Teil C, Seiten 274, 275, 286, 287, 326, 327, 510 und 511**

(73) Patentinhaber: **BOEHRINGER MANNHEIM GMBH**
**Sandhofer Strasse 116**
**D-6800 Mannheim 31 (DE)**

(72) Erfinder: **Trasch, Heinz-Friedrich, Dr.rer.nat.**
**Wissmannstrasse 14**
**D-6700 Ludwigshafen (DE)**
Erfinder: **Endres, Erwin**
**Breite-Strasse 95**
**D-6700 Ludwigshafen (DE)**
Erfinder: **Trost, Andreas**
**Scheffelstrasse 26**
**D-6832 Hockenheim (DE)**
Erfinder: **Rittersdorf, Walter, Dr.rer.nat.**
**Kasseler Strasse 6**
**D-6800 Mannheim 31 (DE)**

(56) Entgegenhaltungen:
**Colour-Index, 3rd Edition, Vol. 4, Seiten 4019 und 4042**

**Handbuch der Plasttechnik VEB-Verlag für Grundstoffindustrie (gutachtlich), Seiten 335 bis 336**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 133 895 B1

Courier Press, Leamington Spa, England.

**Beschreibung**

Klinisch chemische Analysenverfahren sind in den letzten Jahren in zwei Richtungen hin entwickelt worden, die beide unter dem Gesichtspunkt der Verminderung des Kostendrucks zu sehen sind. Die eine Richtung ist die Automatisation und damit die Rationalisierung der Analysenverfahren mit denen in kürzester Zeit große Zahlen von Proben und Parametern in Zentrallaboratorien untersucht wurden. Die andere Richtung ist die Vereinfachung und Beschleunigung von Analysenmethoden, die der Arzt bei der direkten Betreuung seiner Patienten einsetzt. Speziell solche Verfahren, bei denen praktisch in der Gegenwart des Patienten die Ergebnisse erhalten werden können, sind für die ambulante Betreuung aus Kostengründen von elementarem Interesse, da dadurch Wartezeiten drastisch vermindert werden und Ausfallzeiten, die durch Wiedereinbestellung des Patienten, nach Vorliegen des Analysenergebnisses, entstehen, praktisch ausgeschieden werden. Für die schnelle und einfachste Analyse von Einzelproben oder kleinen Serien haben Teststreifen, bei denen die Reagenzien in einer Matrix aus Papier oder Kunststoff enthalten sind und die Probe direkt auf diese Matrix aufgebracht wird, eine überragende Bedeutung gewonnen, zumal mit der Einführung reflexionsphotometrischer Verfahren auch quantitative Auswertungen mit hervorragender Präzision und Richtigkeit möglich gemacht wurden.

Die bisher im Handel befindlichen Teststreifen zur Bestimmung der Inhaltsstoffe des Blutes haben aber folgende Nachteile: Die meisten Verfahren werden durch die Erythrozyten gestört. Der Anweder muß deshalb vor der Analyse aus dem Vollblut das Serum oder Plasma durch Zentrifugieren abtrennen. Dies ist jedoch besonders bei kleinen Probemengen problematisch. Nur wenige Teste, bei denen die Reagenzschicht selbst (DE—A—15 98 153) oder eine vorgeschaltete Membran semipermeabel ist und die Erythrozyten zurückhält, können direkt mit Vollblut beaufschlagt werden. Bei diesen Tests muß aber der rote Blutfarbstoff, vor der reflexionsphotometrischen Vermessung, durch Abwaschen oder Abwischen entfernt werden. Bei großen Molekülen, z.B. Enzymen, kann diese Methode nicht angewandt werden, da diese ebenfalls durch die semipermeable Schicht zurückgehalten werden.

Es hat nicht an Bemühungen gefehlt, Systeme zu entwickeln, bei denen direkt mit Vollblut gearbeitet werden kann und die Abtrennung der Erythrozyten bzw. des Hämoglobins und damit die Gewinnung des Plasmas im System selbst geschieht. Mehrschichtige Testvorrichtungen für Blut, bei denen eine oder mehrere dieser Schichten als Filter wirken, die die korpuskulären Bestandteile des Blutes zurückhalten, sind z.B. aus der DE—AS—2 222 951 oder der US—P—4 144 306 bekannt. Da für die Abtrennung der Erythrozyten jedoch Membranfilter mit Poren von 1—3 µm eingesetzt werden müssen, verstopfen diese leicht und behindern den Durchtritt des Plasmas, welches dadurch langsam und ungleichmäßig in die Reaktionsschicht eintritt. Ein Fortschritt wurde durch den Einsatz von speziellen Glasfaserfiltern erzielt, wie sie in der DE—OS—30 29 579.5 beschrieben sind. Insbesondere zur Herstellung von Teststreifen zur Analyse von Blut lassen sich solche Glasfaserfilter gut verwenden. Die partikulären Blutbestandteile werden dabei vom Plasma auf einfachste Weise abgetrennt, indem man das Vollblut auf ein Glasfaservlies aufträgt, welches die Erythrozyten zurückhält und das Plasma über ein kapillaraktives Vlies (Transportvlies) absaugt und zu der Reagenzschicht transportiert, in der die Nachweisreaktion abläuft.

Diese Systeme haben sich gut bewährt, sie haben jedoch auch Nachteile. Das Totvolumen der Erythrozytenrückhaltezone ist dem Testaufbau und der Trennkapazität entsprechend relativ hoch. Das Verhältnis von abgetrenntem Plasma zu dosiertem Blut ist daher ungünstig und wird bei Blutproben mit höheren Hämatokritwerten noch verschlechtert. Es kommt daher mitunter vor, daß das Abtrennsystem die Menge der abzutrennenden Erythrozyten nicht bewältigt, so daß Blutfarbstoff zur Reaktionszone gelangt und dort Störungen hervorruft.

In den US—P—3 552 925 und 3 552 928 sind anorganische Salze bzw. Aminosäuren beschrieben, die geeignet sein sollen, Blut so weit zu koagulieren, daß es in einem normalen Filterpapier zurückgehalten wird. Die verwendeten Konzentrationen der Salze (1 Molar!) und Aminosäuren (5%) bewirken nur eine relativ schwache Trennung von Erythrozyten und Plasma, aber bereits eine erhebliche Hämolyse, so daß sie nicht zur Plasmagewinnung in Teststreifen geeignet sind.

Es stellte sich deshalb die Aufgabe. "Rückhaltesubstrate" zu finden, die in Konzentrationen, bei denen noch keine Hämolyse eintritt, eine starke Koagulation des Blutes bewirken, so daß die korpuskulären Bestandteile in einem Papier- oder Glasfaservlies wirksam zurückgehalten und vom Plasma getrennt werden.

Es wurde nun überraschend gefunden, daß diese Aufgabe dadurch gelöst werden kann, daß man das zu untersuchende Blut mit "Rückhalte-Substraten" in Berührung bringt, die zwei starke polare Gruppen enthalten, die durch eine als "Spacer" dienende unpolare Brücke verbunden sind, die 2—20 Kohlenstoff- oder Stickstoffatome enthält. Es wird vermutet, daß diese Substrate in der Lage sind, die Polarität der Erythrozytenoberfläche zu ändern und diese dadurch zur Koagulation zu veranlassen. Die Bildung von Agglomeraten im Blut durch den Zusatz der "Rückhalte-Substrate" ist mikroskopisch leicht zu beobachten.

Als Erythrozyten-Rückhaltesubstrate eignen sich Verbindungen der folgenden Formel

$$R_1—Sp—R_2 \qquad (I)$$

in der Sp eine als Spacer dienende Brücke,

$R_1$ eine stark polare Gruppe darstellt und

$R_2$ ebenfalls eine stark polare Gruppe darstellt oder, wenn Sp eine elektronenleitende Gruppe darstellt, auch eine polarisierbare Gruppe bedeutet.

Die Substituenten $R_1$ und $R_2$ sind dabei gleich oder verschieden und bedeuten $SO_3H$, $CO_2H$, $NH_2$ oder OH, insbesondere in Form ihrer Ionen, wobei $R_2$ auch eine $—NO_2$, $—SO_2NH—$, —CONH— Gruppe oder Halogen- oder Alkoxygruppe darstellen kann.

Sp bedeutet bevorzugt ein aromatische oder heteroaromatisches Ringsystem aus mindestens zwei Ringen, die entweder anneliert oder direkt, oder über —CH=CH—, —N=N—, $—CR_3{=}N—$, $—NR_3—CO—NR_4—$, $—CR_3R_4—$ verbunden sind, wobei $R_3$ und $R_4$ Wasserstoff oder eine Alkylgruppe darstellen. Besonders bevorzugt sind solche Ringsysteme, bei denen die Substituenten $R_1$ und $R_2$ an verschiedenen Ringen sitzen und gegebenenfalls noch weitere Substituenten der Bedeutung von $R_1$ sowie $NO_2$, Halogen, Alkoxy, Alkyl, $SO_2$, $NR_3$ und $CONR_3$ enthalten sein können. Weiterhin bevorzugt sind aliphatische Diamine mit 2—10 C-Atomen. Die Gruppen $R_1$ und $R_2$ liegen bevorzugt in ionisierter Form vor, da die entsprechenden Verbindungen einerseits besser in Wasser löslich sind und andererseits die höhere Polarität der Ionen die Rückhalteeigenschaften verbessert. Alkyl und Alkoxygruppen im Sinne dieser Erfindung sind geradkettige, verzweigte oder cyclische Reste mit 1—10, vorzugsweise 1—6 C-Atomen, insbesondere die Methyl- und Ethylgruppe.

Die Rückhalte-Substrate sind überwiegend Farbstoffe und gehören den verschiedensten Substanzklassen an:

Säurefarbstoffe, basische Farbstoffe, Direktfarbstoffe, Beizenfarbstoffe, Reaktivfarbstoffe, optische Aufheller, aber auch Verbindungen ohne Farbstoffcharakter, wie z.B. aliphatische Diamine.

Demgemäß ist Gegenstand der Erfindung ein Verfahren zum Abtrennen von Erythrozyten aus Blut, dadurch gekennzeichnet, daß man das Blut mit einer Substanz aus der Gruppe der Verbindungen der allgemeinen Formel

$$R_1—Sp—R_2 \qquad (I)$$

in der

$R_1$ und $R_2$ gleich oder verschieden sind und $—SO_3H$, $—CO_2H$, $—NH_2$ oder —OH, insbesondere in Form ihrer Ionen,

$R_2$ auch eine Nitro-, Sulfonamid-, Carbonsäureamid-Gruppe oder Halogen- oder Alkoxygruppe und

Sp ein aromatisches oder heteroaromatisches Ringsystem aus mindestens zwei Ringen, die entweder anneliert oder direkt oder über —CH=CH—, —N=N—, $—CR_3{=}N—$, $—NR_3—CO—NR_4—$, $—CR_3R_4—$ verbunden sind, wobei $R_3$ und $R_4$ Wasserstoff oder eine Alkylgruppe bedeuten und die Ringsysteme gegebenenfalls auch noch weitere Substituenten tragen können oder

wenn $R_1$ und $R_2$ zugleich $—NH_2$ bedeuten, auch eine Alkylengruppe mit 2—10 C-Atomen darstellt, und

Primazingelb GRL®, Levafixbrilliantgelb E3G®, Levafix E-R2®, Levafixgoldgelb EG®, Blankophor REU®, Blankophor RA®, Blankophor BE®, Blankophor BA 267®, Blankophor BBH®, Blankophor RPA®, Blankophor BBU®, Tinopal BV® (C.I. 40630), Tinopal UP®, Uvitex NB®, Uvitex RT®, Uvitex CF®, Anthrachinondisulfonsäure-2,6 (Na-Salz), 2 - Ethoxy - 6,9 - diaminoacridin, Primazin - Brilliantgelb 3-GL®, Remazolgelb FG®, Remazolgelb G®, Remazolgelb GNL®, Remazolgelb FGH®, Remazolbrilliantgelb GL®, Solidarzolbrilliantgelb 4G®, Säuregelb 38® (C.I. 25135), Säuregelb 40® (C.I. 18950), Säuregelb 65® (C.I. 14170), Säuregelb 76® (C.I. 18850), Säuregelb 99® (C.I. 13900), Direktgelb 8® (C.I. 13920), Direktgelb 27® (C.I. 13950), Direktgelb 62® (C.I. 36900), Basic Yellow 11® (C.I. 48055), Thiazolgelb® (C.I. 19540) und Papiergelb RF® (C.I. 40000).

In Kontakt bringt, gleichzeitig oder anschließend durch eine Filterschicht aus Glasfasern, Zellulose, Kunststoff oder Asbestfasern filtriert und das abgetrennte Plasma gewinnt.

Desweiteren ist Gegenstand der Erfindung die Verwendung von Substanzen aus der Gruppe der Verbindungen der allgemeinen Formel

$$R_1—Sp—R_2 \qquad (I)$$

in der

$R_1$ und $R_2$ gleich oder verschieden sind und $—SO_3H$, $—CO_2H$, $—NH_2$ oder —OH, insbesondere in Form ihrer Ionen,

$R_2$ auch eine Nitro-, Sulfonamid-, Carbonsäureamid-Gruppe oder Halogen- oder Alkoxygruppe und

Sp ein aromatisches oder heteroaromatisches Ringsystem aus mindestens zwei Ringen, die entweder anneliert oder direkt oder über —CH=CH—, —N=N—, $—CR_3{=}N—$, $—NR_3—CO—NR_4—$, $—CR_3R_4—$ verbunden sind, wobei $R_3$ und $R_4$ Wasserstoff oder eine Alkylgruppe bedeuten und die Ringsysteme gegebenenfalls auch noch weitere Substituenten tragen können oder

wenn $R_1$ und $R_2$ zugleich $—NH_2$ bedeuten, auch eine Alkylengruppe mit 2—10 C-Atomen darstellt, und

Primazingelb GRL®, Levafixbrilliantgelb E3G®, Levafix E-R2®, Levafixgoldgelb EG®, Blankophor REU®, Blankophor RA®, Blankophor BE®, Blankophor BA 267®, Blankophor BBH®, Blankophor RPA®, Blankophor BBU®, Tinopal BV® (C.I. 40630), Tinopal UP®, Uvitex NB®, Uvitex RT®, Uvitex CF®, Anthrachinondisulfonsäure - 2,6 (Na-Salz), 2 - Ethoxy - 6,9 - diaminoacridin, Primazin - Brilliantgelb 3-GL®, Remazolgelb FG®, Remazolgelb G®, Remazolgelb GNL®, Remazolgelb FGH®, Remazolbrilliantgelb

GL®, Solidazolbrilliantgelb 4G®, Säuregelb 38® (C.I. 25135), Säuregelb 40® (C.I. 18950), Säuregelb 65® (C.I. 14170), Säuregelb 76® (C.I. 18850), Säuregelb 99® (C.I. 13900), Direktgelb 8® (C.I. 13920), Direktgelb 27® (C.I. 13950), Direktgelb 62® (C.I. 36900), Basic Yellow 11® (C.I. 48055), Thiazolgelb® (C.I. 19540) und Papiergelb RF® (C.I. 40000) zum Abtrennen von Erythrozyten aus Blut, sowie ein Mittel, das diese Verbindungen in trägergebundener Form enthält.

Naturgemäß sind nicht alle Rückhalte-Substrate für jeden Test gleichermaßen gut geeignet. So bewirken manche Stoffe partielle Hämolyse, die bei manchen chemischen Reaktionen stören können. Ebenso können manche Rückhalte-Substrate unerwünschte Wechselwirkungen mit Enzymen eingehen. Beide Effekte sind nicht unmittelbar aus der Struktur herleitbar, solche Stoffe sind aber durch einfache Versuche leicht zu ermitteln. Sie können darum für den entsprechenden Test nicht eingesetzt werden. Die große Auswahl an Rückhalte-Substraten beinhaltet jedoch für jeden Test eine genügende Anzahl an geeigneten Vertretern. Naturgemäß muß die Auswähl der Rückhalte-Substrate so erfolgen, daß deren Eigenfarbe die Reaktionsfarbe des Testes nicht verfälscht, was durch die große Variabilität der Verbindungsklassen ermöglicht wird. Die Rückhalte-Substrate entfalten ihre Wirksamkeit nicht nur in Lösung, sondern auch dann, wenn sie fest mit dem Träger verbunden sind, z.B. bei der Verwendung von Direktfarbstoffen auf cellulosehaltigen Trägern. Die oben genannten Reaktivfarbstoffe können zwar mit den Trägern reagieren, sie müssen dies aber nicht, denn ihre Wirksamkeit ist auch dann gegeben, wenn sie von der Faser abwaschbar sind.

Die Eignung eines Substrates läßt sich in einem einfachen Screening-Versuch ermitteln, bei dem entweder das Blut mit 0,5—1% des Substrates vermischt wird und danach mit einer Kapillare auf ein "Rückhalte-Matrix", z.B. ein Filterpapier oder Glasfaservlies, aufgetragen wird, wo sich um die Auftragstelle herum die Erythrozyten abscheiden und außen ein Ring von klarem Plasma ausbreitet. Bei brauchbaren Substraten ist dieser äußere Ring mindestens zweimal, vorzugsweise 3 mal so groß wie die erythrozytenhaltige Zone. Für Reihenteste besser geeignet ist ein anderer Versuch, bei dem 1—5 %-ige Lösungen der Substrate auf ein dünnes Papier imprägniert werden, welches auf ein als "Rückhalte-Zone bzw. Filter" dienendes größeres Vlies oder Papier gelegt wird. Wenn nun Vollblut auf das erste Papier aufgetragen wird, reichert es sich mit dem Substrat an, so daß die Erythrozyten in der Rückhalte-Zone abgeschieden werden und sich eine klare Plasmafront ausbreitet.

Der Einsatz der erfindungsgemäßen Rückhalte-Substrate kann nach verschiedensten Varianten erfolgen. Die Art des Einsatzes hängt von dem Aufbau bzw. Ablaufschema des Analysensystems, in den man die Rückhalte-Substrate einsetzen will, ab. Eine Reihe von möglichen Ausführungsformen ist in der DE—OS—30 29 579.5 beschrieben. Weitere Abwandlungen kann der Fachmann unschwer entwickeln.

Bevorzugt wird ein Analysensystem gemäß Fig. 1. Auf einen Folienstreifen 1 ist ein Streifen 5 eines saugfähigen Materials aufgebracht, über einem Teil dieses Materials 5b befindet sich ein Trockenreagenzträger 8, dessen Reagenzzone 6 mit Reagenzien zum Nachweis von z.B. Glucose, Harnstoff, Harnsäure, GOT, gamma-GT oder anderen Substraten oder Enzymen imprägniert ist. Der Träger ist mit der Klebestelle 15 an der Folie 1 so befestigt, daß 6 durch Druck von oben mit 5b in Kontakt gebracht werden kann. Über dem anderen Teil 5a des saugfähigen Materials 5 befindet sich die Erythrozytenrückhaltezone 3, die die Rückhalte-Substrate enthält. Die Blutprobe wird auf das Rückhaltesystem 3 pipettiert. Der das Nachweisverfahren störende rote Blutfarbstoff, bzw. die Erythrozyten, werden in 3 und 5a zurückgehalten, das Plasma gelangt durch das saugfähige Transportmaterial 5 in den Bezirk 5b unter die Reagenzzone 6. Diese wird nachdem sich der Bezirk 5b mit Plasma gefüllt hat, angedrückt und dadurch mit Plasma benetzt; die Nachweisreaktion läuft ab und die entstandene Farbe wird visuell beurteilt oder remissionsphotometrisch vermessen.

Das Rückhalte-Substrat kann entweder direkt in 3 enthalten sein oder darüber auf einem nicht dargestellten zusätzlichen saugfähigen oder nicht saugfähigen durchlässigen Träger 2 angebracht sein.

Als saugfähige Materialien für die Rückhaltezone 3, die mit den Rückhalte-Substraten ausgerüstet werden, können prinzipiell alle saugfähigen, porösen, für Flüssigkeit durchlässigen Träger bzw. Filter benutzt werden, wie z.B. Papiere, Vliese, Gele, Gewebe usw. aus z.B. Cellulose Wolle, Glasfaser, Asbest, synthetischen Fasern, Polymeren oder Gemischen derselben, die geeignet sind, die agglomerisierten Erythrozyten abzutrennen, das heißt Materialien die Porengrößen, die größer sind als einzelne Erythrozyten, das heißt ungefähr 10—100 µ, aufweisen.

Nichtsaugfähige Materialien für den zusätzlichen Substratträger 2 sind z.B. Netze aus groben Fasern. Das Beladen der Träger mit den Rückhalte-Substraten geschieht durch einfaches Imprägnieren derselben aus wäßrigen oder organischen Lösungen. Man kann auch zu den Lösungen filmbildende oder haftvermittelnde Substanzen, wie z.B. Gelatine, Alginate und andere Naturprodukte sowie wasserlösliche synthetische Verbindungen, wie z.B. Polyvinylpyrrolidone usw. einsetzen, ferner Puffer, oberflächenaktive Substanzen oder andere Hilfsmittel, sofern diese die Hämolyse des Blutes nicht fördern und die anzuschließende Testreaktion nicht stören.

Das Transportmaterial 5 ist vorzugsweise ein Glasfaservlies, da dies mit dem Plasma nur geringe Wechselwirkungen aufweist, jedoch kann dies auch durch andere kapillaraktive Materialien, z.B. Filterpapier, ersetzt werden, sofern diese genügend Plasma an die Reagenzzone 6 abgeben. Weitere geeignete Materialien sind durch Versuche unschwer zu ermitteln.

Von besonderem Vorteil ist es, die Rückhalte-Substrate schon vorher dem Blut beizufügen, vorzugsweise während der Blutabnahme. Die Blutabnahmevorrichtung enthält dann die Rückhalte-

Substrate in fester Form, beispielsweise an der Innenwand der Abnahmevorrichtung oder gelöst in physiologischer Kochsalz-Lösung in einem geeigneter Behälter. Durch die längere Kontaktzeit mit dem Rückhalte-Substrat verstärkt sich die Koagulation der Erythrozyten so, daß eine kleinere Rückhaltezone 3 ausreicht und die Plasmaausbeute verbessert wird.

In den folgenden Beispielen soll die Erfindung näher erläutert werden:

Beispiel 1
Herstellung eines Teststreifens zum Nachweis von Glucose im Blut

| | |
|---|---|
| 35 KU | Glucoseoxidase |
| 200 KU | Peroxidase |
| 15 ml | 0,5 m Phosphatpuffer pH 5 |
| 0,3 g | Na-Alginat |
| 25 g | Dispersion aus einem Mischpolymeren von Vinylacetat und Vinylpropionat (50 %ig in Wasser) |
| 0,5 g | Tetramethylbenzidin (3,3',5,5') |
| 0,2 g | Phenylsemicarbazid |
| 1 g | Dioctylnatriumsulfosuccinat |
| 6 ml | Methoxyäthanol |
| 20 g | Titandioxid |
| 35 ml | Wasser |

werden zu einer homogenen Masse verarbeitet und mit 0,1 mm Spaltbreite auf ein 250 µ dickes multifiles Polyamid-Gewebe (2F 131 Schweizer Seidengaze-Fabrik) beschichtet und getrocknet. Die so erhaltene Reagenzzone 6 mit dem Reagenzfilm 6a wird danach mit einer durchsichtigen Abdeckfolie 7 verbunden, so daß die Abdeckfolie auf dem Reagenzfilm 6a aufliegt. Anschließend wird ein 1 cm breiter Streifen dieses beschichteten Gewebes 6 mit der Gewebeseite 6b nach unten entsprechend Fig. 2 über eine Klebestelle 15 auf einem Plastikstreifen 1 befestigt, auf dem bereits ein 15 mm breites Glasfaservlies 5, Dicke 0,25 mm, Flächengewicht ca. 25 g/m$^2$ aufgebracht ist, so daß das freie Ende des beschichteten Gewebes noch 6 mm über das Vlies reicht (Zone 5b). Auf dem Glasfaservlies ist das Abtrennsystem aufgebracht. Es besteht aus einem Glasfaservlies 3, Dicke 1 mm und einem mit 5%iger wässriger Lösung von Remazolgelb GNL$^R$ imprägnierten Substratträger 2 (Schablonenpapier, Firma Schöller und Hösch, Flächengewicht 12 g/m$^2$), die durch das Nylonschutznetz 4 mit dem Plastikstreifen 1 verbunden sind. Bringt man nun 30 µl Vollblut auf das Nylonnetz 4, so durchdringt innerhalb kurzer Zeit das Blut das gesamte Abtrennsystem, vermischt sich mit dem Rückhalte-Substrat und führt zur Agglomeration der Erythrozyten. Die agglomerierten Erythrozyten werden zurückgehalten, das Plasma wandert auf dem Transportvlies 5 unter das mit dem Reagenzfilm 6a beschichtete Gewebe 6b.

Durch Drücken auf die Abdeckfolie 7 kommt nun die Reagenzzone 6 über die Gewebeseite 6b mit dem abgetrennten Plasma in Berührung und wird gleichmäßig durchfeuchtet. Die im Plasma enthaltene Glucose reagiert innerhalb von 1 bis 2 Minuten in Abhängigkeit von ihrer Konzentration unter Entwicklung einer mehr oder weniger kräftigen Blaufärbung, die bei 630 nm remissionsphotometrisch vermessen wird.

Läßt man im beschriebenen Beispiel das mit Remazolgelb GNL$^R$ imprägnierte Schablonenpapier weg, so wandern die Erythrozyten so weit auf dem Transportvlies, daß sie bei der remissionsphotometrischen Vermessung des Testfeldes zu Störungen führen.

| mg/dl Glucose | % Remission |
|---|---|
| 0 | 75 |
| 80 | 62 |
| 150 | 44 |
| 210 | 40 |
| 300 | 35 |
| 400 | 30 |

Beispiel 2
Herstellung eines Teststreifens zum Nachweis von Bilirubin im Blut

| | |
|---|---|
| 0,2 g | 2-Methoxy-4-nitrobenzoldiazoniumtetrafluoroborat |
| 1,5 g | Metaphosphorsäure |
| 1,5 g | Diphenylphosphorsäure |
| 0,2 g | Dioctylnatriumsulfosuccinat |
| 5 g | Kieselgel |
| 1 g | Cellulose |
| 7,5 g | Polyvinylidenchlorid-Dispersion (Diofan 217 D, BASF) (40 %-ig in Wasser) |
| 15 g | Quellmittel (Bentone EW, National Lead) 2,5 %-ig in Wasser |

werden zu einer homogenen Masse verarbeitet und mit 0,2 mm Spaltbreite auf ein 200 µm dickes multifiles Polyester-Gewebe (2 F 777 Schweizer Seidengaze-Fabrik) beschichtet und getrocknet.

Der so erhaltene beschichtete Träger wird, wie in Beispiel 1 beschrieben, zu einem Teststreifen verarbeitet, jedoch war der Substratträger 2 mit einer 5 %-igen Lösung der Naphthalindisulfonsäure-2,6 (Na-salz) imprägniert. Die Reaktion mit bilirubinhaltigem Blut geschieht wie in Beispiel 1 und ergibt nach 60 Sekunden Reaktionszeit eine ausgezeichnete Abstufung über den gesamten relevanten Konzentrationsbereich.

Beispiel 3
Herstellung eines Teststreifens zum Nachweis von Cholesterin im Blut

| | |
|---|---|
| 2,5 KU | Cholesterinoxidase |
| 1,5 KU | Cholesterinesterase |
| 50 KU | Peroxidase |
| 10 mg | Gallussäure |
| 0,5 g | Tetramethylbenzidin (3,3',5,5') |
| 0,3 g | Dioctylnatriumsulfosuccinat |
| 1,5 ml | Aceton |
| 6,5 g | Dispersion aus einem Mischpolymeren von Vinylacetat und Vinylpropionat (50 %ig in Wasser) |
| 5 g | Titandioxid |
| 10 g | Cellulose |
| 15 ml | Phosphatpuffer 0,5 M, pH 7 |
| 20 ml | Wasser |

werden zu einer homogenen Masse verarbeitet und mit 0,15 mm Spaltbreite auf eine 140 µm dicke Polycarbonatfolie beschichtet und getrocknet.

Der so beschichtete Reagenzträger 8 wird wie in Beispiel 1 beschrieben zu einem Teststreifen verarbeitet, wobei die Abdeckfolie entfällt und die beschichtete Folie, mit dem Reagenzfilm nach unten angeordnet, das Gewebe ersetzt. Im Abtrennsystem wird das Glasfaservlies 3 durch ein weiteres Schablonenpapierchen ersetzt (Fig. 3).

Für den Nachweis von Cholesterin in Blut wurden zwei Teebeutelpapierchen (Schöller und Hösch, Flächengewicht 12 g/m$^2$) mit 5% Remazolgelb FGH$^R$ in Wasser imprägniert, eingesetzt.

Die Reaktion mit cholesterinhaltigem Blut geschieht durch Andrücken der beschichteten Folie auf das mit Plasma gefüllte Transportvlies. Das abgetrennte Plasma gelangt so auf den Reagenzfilm, durchfeuchtet ihn und ergibt nach 100 Sekunden Reaktionszeit eine ausgezeichnete Abstufung über den gesamten relevanten Konzentrationsbereich.

Beispiel 4
Herstellung eines Teststreifens zum Nachweis von Triglyceriden im Blut

| | |
|---|---|
| 50 KU | Peroxidase |
| 20 KU | Cholesterinesterase |
| 50 KU | Glycerinkinase |
| 10 KU | Glycerophosphat-Oxidase |
| 20 g | Dispersion aus einem Mischpolymeren von Vinylacetat und Vinylpropionat (50 %ig in Wasser) |
| 20 g | Cellulose |
| 0,2 g | Na-Alginat |
| 10 g | Titandioxid |
| 0,68 g | Tetramethylbenzidin (3,3',5,5') |
| 0,30 g | Dioctylnatriumsulfosuccinat |
| 1,5 ml | Aceton |
| 25 ml | Phosphatpuffer 0,2 M, pH 7,8 |
| 10 ml | Wasser |
| 0,2 g | Adenosintriphosphat |

werden zu einer homogenen Masse verarbeitet und mit 0,2 mm Spaltbreite auf eine 140 µm dicke Polycarbonatfolie beschichtet und getrocknet. Der so erhaltene beschichtete Träger wird, wie in Beispiel 3 beschrieben, zu einem Teststreifen verarbeitet. Die Reaktion mit triglyceridhaltigem Blut geschah wie in Beispiel 3 und ergab nach 120 Sekunden Reaktionszeit eine ausgezeichnete Abstufung über den gesamten relevanten Konzentrationsbereich.

Als Transportvlies 5 wurde Filterpapier (Firma Schleicher und Schüll, Nr. 0858) verwendet. Die Rückhaltezone 3 besteht aus dem Mischfaservlies (VS 532, Firma Binzer), imprägniert mit 5% Naphtholgelb S$^R$ in Wasser.

Beispiel 5
Herstellung eines Teststreifens zum Nachweis von Harnsäure im Blut

| | |
|---|---|
| 40 KU | Peroxidase |
| 1 KU | Uricase |
| 18 g | Dispersion aus einem Mischpolymeren von Vinylacetat und Vinylpropionat (50 %ig in Wasser) |
| 0,25 g | Na-Alginat |
| 0,5 g | nichtionogenes Netzmittel |
| 0,05 g | EDTA-$Na_2$ |
| 20 g | Kieselgur |
| 20 ml | Phosphatpuffer 0,2 m, pH 7 |
| 0,4 g | Primaquin-diphosphat |
| 18 ml | Wasser |

werden zu einer homogenen Masse verarbeitet und mit 0,2 mm Spaltbreite auf eine 150 µm dicke Polyesterfolie beschichtet und getrocknet.

Indikatorpapier
mit

| | |
|---|---|
| 0,2 g | 4-Aminoantipyrin |
| 0,2 g | nichtionogenes Netzmittel |
| in 50 ml | Wasser |

wird ein dünnes Filterpapier (597 NF-Ind., Schleicher und Schüll) getränkt und getrocknet.

Es werden Teststreifen, wie in Beispiel 3 beschrieben, hergestellt, die zusätzlich zwischen Transportvlies und Reagenzfilmunterseite eine Lage Aminoantipyrinpapier 12 erhalten (Fig. 4).

Das harnsäurehaltige Blut wurde mit einer Abnahmevorrichtung gewonnen, die bei der Entnahme das Blut mit Solidazolbrilliantgelb 4G[R] vermischt. Das so behandelte Blut wird auf das Schutznetz 4 des Teststreifens gebracht und ergab nach 120 Sekunden Reaktionszeit eine ausgezeichnete Abstufung über den gesamten relevanten Konzentrationsbereich.

Beispiel 6
Herstellung eines Teststreifens zum Nachweis von gamma-Glutamyltransferase im Blut

| | |
|---|---|
| 1,0 g | N-Methylanthranilsäure |
| 2,5 g | Glycylglycin |
| 0,85 g | EDTA-$Na_2$ |
| 0,2 g | Gluatamyl-p-phenylendiamin-3-carbonsäure |
| 20 g | Dispersion aus einem Mischpolymeren von Vinylacetat und Vinylpropionat (50 %ig in Wasser) |
| 0,2 g | Na-Alginat |
| 0,35 g | Dioctylnatriumsulfosuccinat |
| 1,0 ml | Methanol |
| 5 g | Titandioxid |
| 8 g | Cellulose |
| 15 ml | Tris-Puffer, pH 7,6 |
| 15 ml | Wasser |

werden zu einer homogenen Masse verarbeitet und mit 0,15 mm Spaltbreite auf ein 250 µm dickes multifiles Polyamid-Gewebe (1093 Verseidag-Industrie-Textilien GmbH) beschichtet und getrocknet.

Mit 250 mmol/l $K_3$ $Fe(CN)_6$ wird ein Teebeutelpapier (Fa. Schöller und Hösch, 12 g/$cm^2$ Flächengewicht) imprägniert und 5 Minuten bei 30°C getrocknet. Es werden Teststreifen, wie in Fig. 3 beschrieben, hergestellt. Die Rückhaltezone 3 besteht aus einem Teebeutelpapierschen 2, imprägniert mit dem Na-salz der Anthrachinondisulfonsäure-2,6 (5% in Wasser) als Rückhalte-Substrat und ein Teebeutelpapierchen 2 imprägniert mit $K_3$ $Fe(CN)_6$ (5% in Wasser).

Die Reaktion mit gamma-GT-haltigem Blut geschah wie in Beispiel 1 und ergab nach 120 Sekunden Reaktionszeit eine ausgezeichnete Abstufung über den gesamten relevanten Konzentrationsbereich.

Fig. 5 zeigt eine weitere Teststreifenvariante, bei der das Abtrennsystem, wie in den vorigen Beispielen beschrieben, entfällt, dafür das Transportpapier 5 mit dem Rückhalte-Substrat Remazolbrilliantgelb GL[R] imprägniert ist. Beim Einsatz von vorbehandeltem Blut (z.B. mit 1% Direktgelb 62 oder Thiazolgelb G[R]) kann gegebenenfalls die gleiche Teststreifenanordnung wie Fig. 5 zeigt benutzt werden, wobei das Transportsystem (Papier, Glasfaservliese...) nicht imprägniert zu sein braucht.

Beispiel 7

Herstellung eines Teststreifens zum Nachweis von Glutamat-Pyruvat-Transaminase im Blut

A) Enzympapier

0,2 Mol/l Morpholinethansulfonsäure/Kaliumhydroxid, pH 6,5
0,8 Mol/l Alanin
$0{,}5\times10^{-3}$ Mol/l Thiaminpyrophosphat
$10\times10^{-3}$ Mol/l Mg-chlorid
$1\times10^{-3}$ Mol/l Kaliumhydrogenphosphat
20 KU/l Peroxidase
200 KU/l Pyruvatoxidase

Mit dieser Lösung wird ein Teebeutelpapier der Firma Schöller und Hösch, 12 g/m² Flächengewicht imprägniert und 5 Minuten bei 30°C getrocknet.

B) Indikatorpapier

$10\times10^{-3}$ Mol/l 4 - (4 - Dimethylamino - phenyl) - 2 - (3,5 - Di - methoxy - 4 - hydroxyphenyl) - imidazol
$18\times10^{-3}$ Mol/l alpha-Ketoglutarsäure gelöst in 0,1 Mol/l Salzsäure.

Mit dieser Lösung wird ein Teebeutelpapierchen der Firma Schöller und Hösch, 12 g/m² Flächengewicht imprägniert und 5 Minuten bei 30°C getrocknet.

Die Verarbeitung zu einem Teststreifen erfolgt wie in Beispiel 1, wobei die Reagenzzone 6 durch die beiden imprägnierten Papiere 12 und 13 ersetzt wurde (Fig. 6). Die Reaktion mit GPT-haltigem Blut geschieht durch Andrücken der Abdeckfolie 7 auf das mit Plasma gefüllte Transportvlies 5. Plasma wandert nun durch das Indikatorpapier 12 in das Enzympapier 13 und ergibt innerhalb von 2 Minuten eine ausgezeichnete Abstufung über den gesamten relevanten Konzentrationsbereich.

Als Rückhaltezone 3 wurde ein mit "Acid Yellow 25" imprägniertes Schablonenpapier (Firma Schöller und Hösch, Flächengewicht 12 g/m²) in Kombination mit einem Filterpapier von Schleicher und Schüll, Nr. 1406, verwendet.

Beispiel 8

Einfache Vorrichtung zur Eignungsprüfung von Rückhaltesubstraten

Wie in der Fig. 7 dargestellt ist, wird auf einem Plastikstreifen 1 von 10 cm Länge und 6 mm Breite ein 15 mm langes Glasfasertransportvlies 5 (Flächengewicht 25 g/m²) geklebt. An einem Ende des Transportvlieses werden als Rückhaltezone 3 zwei mit Rückhaltesubstraten imprägnierte, 6×6 mm große Schablonenpapierchen Schöller & Hösch, Flächengewicht 12 g/m²) mittels eines Schutznetzes 4 so befestigt, daß die imprägnierten Schablonenpapierchen vollständig bedeckt sind. Bringt man nun 30 µm Vollblut auf das Schutznetz, dann durchdringt das Blut die beiden Substratpapierchen und vermischt sich mit dem Rückhaltesubstrat. Das Blut tritt dann in das Transportvlies ein, in dem es kapillar wandert, wobei die Erythrozyten zurückgehalten werden. Geeignete Rückhaltesubstrate liegen dann vor, wenn die Erythrozytenlaufstrecke kürzer ist, als bei der Vergleichsmessung mit nicht imprägnierten Papierchen. Die Laufstrecke wurde 1 Minute nach Auftragen des Vollblutes gemessen, nachdem das Transportvlies ganz mit Plasma gefüllt ist.

Das Schablonenpapier wurde mit 3—5 %igem (W/V) wässrigen Lösungen imprägniert und 30 Minuten bei 35°C getrocknet. Die Länge der Laufstrecke ist im geringen Maße abhängig von eingesetztem Blut. Nachfolgend eine Liste von Substanzen, die für einen Meßbereich von 600—650 nm bei der Reflexionsphotometrie als Rückhaltesubstrate eingesetzt werden können. In Klammern ist, soweit bekannt, jeweils die "colour index no." oder die chemische Bezeichnung hinzugefügt.

| Erythrozytenlaufstrecke im Prüfmodell, in mm | Rückhaltesubstrat |
|---|---|
| 5 | Kontrolle ohne Imprägnierung |
| 3 | Primazingelb GRL$^R$ 1) (reactive yellow 15) |
| 3 | Levafixbrilliantgelb E3G$^R$ 2) (reactive yellow 25) |
| 1,5 | Levafix E-R2$^R$ 2) (reactive yellow 26) |
| 1,5 | Levafixgoldgelb EG$^R$ 2) (reactive yellow 27) |
| 2 | Naphtholgelb S (2,4-Dinitronaphthol-1,7-sulfonsäure-diNa-Salz) |
| 2,5 | Chrysophenin G extra (direct yellow 12) |
| 1,5 | Blankophor G$^R$ 2) (Fluorescent brightener 40) |
| 2 | Blankophor REU$^R$ 2) (Fluorescent brightener 119) |
| 2,5 | Blankophor RA$^R$ 2) (Fluorescent brightener 204) |
| 2,5 | Blankophor R$^R$ 2) (Fluorescent brightener 30) |
| 2 | Blankophor CE$^R$ 2) (Fluorescent brightener 118) |
| 3 | Blankophor BE$^R$ 2) (Fluorescent brightener 115) |

| Erythrozytenlaufstrecke im Prüfmodell, in mm | Rückhaltesubstrat |
|---|---|
| 2,5 | Blankophor BA 267$^R$ 2) (Fluorescent brightener 113) |
| 0,5 | Blankophor BBH$^R$ 2) |
| 0,5 | Blankophor RPA$^R$ 2) (Fluorescent brightener 148) |
| 0,5 | Blankophor BBU$^R$ 2) (Fluorescent brightener 114) |
| 1 | Tinopal BV$^R$ 3) (Fluorescent brightener 1) (C.I. 40630) |
| 3 | Tinopal UP$^R$ 3) (Fluorescent brightener 154) |
| 2 | Uvitex NB$^R$ 4) (Fluorescent brightener 183) |
| 1,5 | Uvitex RT$^R$ 4) (Fluorescent brightener 37) |
| 3 | Uvitex CF$^R$ 4) (Fluorescent brightener 134) |
| 1,5 | Ultraphor WT$^R$ 1) (Fluorescent brightener 48) (C.I. 40640) |
| 1,5 | Naphthalindisulfonsäure-2,6 (Na-Salz) |
| 1,0 | 1-Naphtholdisulfonsäure-3,6 (Na-Salz) |
| 1,0 | Anthrachinondisulfonsäure-2,6 (Na-Salz) |
| 2,5 | Rivanol$^R$ 5) (2-Ethoxy-6,9-diaminoacridin) |
| 1,5 | Chrysoidin HR$^R$ 5) (basic orange 2) (C.I. 11270) |
| 2,5 | Primazinbrilliantgelb 3-GL$^R$ 1) (reactive yellow 37) |
| 0,5 | Remazolgelb FG$^R$ 5) (reactive yellow 42) |
| 0,5 | Remazolgelb G$^R$ 5) (reactive yellow 14) |
| 0,5 | Remazolgelb GNL$^R$ 5) (reactive yellow 23) |
| 0,5 | Remazolgelb FGH$^R$ 5) (reactive yellow 42:1) |
| 0,5 | Remazolbrilliantgelb GL$^R$ 5) (reactive yellow 37) |
| 0,5 | Solidazolbrilliantgelb 4G$^R$ 6) (reactive yellow 40) |
| 2,5 | Brilliantgelb (direct yellow 4) (C.I. 24890) |
| 1,5 | Brilliantsulfaflavine (acid yellow 7) (C.I. 56205) |
| 1,5 | 4,4'-Diaminostilben-2,2'-disulfonsäure (Na-Salz) |
| 1,5 | 4-Nitro-4'-aminostilben-2,2'-disulfonsäure (Na-Salz) |
| 2,5 | 2-(4-Aminophenyl)-6-methyl-benzothiazol-7-sulfonsäure (Na-Salz) |
| 0,5 | Säuregelb 17 (food yellow 5) (C.I. 18965) |
| 0,5 | Säuregelb 23 (Tartrazin) (C.I. 19140) |
| 0,5 | Säuregelb 25 (acid yellow 25) (C.I. 18835) |
| 2,0 | Säuregelb 34 (acid yellow 34) (C.I. 18890) |
| 2,5 | Säuregelb 38 (acid yellow 38; (C.I. 25135) |
| 1,5 | Säuregelb 40 (acid yellow 40) (C.I. 18950) |
| 1,5 | Säuregelb 65 (acid yellow 65) (C.I. 14170) |
| 1 | Säuregelb 76 (acid yellow 76) (C.I. 18850) |
| 1 | Säuregelb 99 (acid yellow 99) (C.I. 13900) |
| 0,5 | Mordant Orange 10 (3-Methyl-5-[4-(4-sulfobenzolazo)-benzolazo]-salizilsäure-Na-Salz) (C.I. 26560) |
| 2,5 | Mordantgelb 3R (5-(p-Nitrobenzolazo)-salizilsäure-Na-Salz) |
| 0,5 | Mordantgelb 7 (5-[p-Sulfobezolazo-]-3-methyl-salizilsäure-Na-Salz) |
| 1,5 | Mordantgelb 10 (5-[p-Sulfobenzolazo-]-3-methyl-salizilsäure-Na-Salz) (C.I. 14010) |
| 0,5 | Mordantgelb 12 (5-[p-Aminobenzolazo-]-3-methyl-salizilsäure-Na-Salz) (C.I. 14045) |
| 1,5 | Direkt Gelb 8 (acid yellow 186) (C.I. 13920) |
| 0,5 | Direkt Gelb 27 (C.I. 13950) |
| 1,5 | Direkt Gelb 29 (C.I. 19556) |
| 0,5 | Direkt Gelb 50 (C.I. 29025) |
| 0,5 | Direkt Gelb 62 (C.I. 36900) |
| 0,5 | Direkt Orange 31 (C.I. 23655) |
| 1 | Basic Yellow 11 (C.I. 48055) |
| 1,5 | Alizaringelb R (mordant orange 1, C.I. 14030) |
| 1,5 | Alizaringelb GG (mordant yellow 1, C.I. 14025) |
| 1 | Thiazolgelb (C.I. 19540) |
| 0,5 | Primulin (direct yellow 59, C.I. 49000) |
| 0,5 | Papiergelb RF (direct yellow 11, C.I. 40000) |
| 0,5 | Farbstoff-Echtgelb (acid yellow 9, C.I. 13015) |
| 2—3 | 1,6-Diaminohexan |
| 2—3 | 1,4-Diaminobutan |
| 2,5—3 | 1,3-Diaminopropan |
| 2—3 | 1,2-Diaminoäthan |
| 0 | 1,6-Diaminohexan×2 HCl |

| Erythrozytenlaufstrecke im Prüfmodell, in mm | Rückhaltesubstrat |
|---|---|
| 0 | 1,4-Diaminobutan×2 HCl |
| 0 | 1,3-Diaminopropan×2 HCl |
| 0 | 1,2-Diaminoethan×2 HCl |
| 5 | 4,4-Dihydroxybiphenyl |
| 5 | 2,3-Diaminonaphthalin |
| 1 | 1,8-Diaminonaphthalin |
| 3,5—4,5 | 1,5-Diaminonaphthalin (90—92%) |
| 4 | 4,4-Dinitrostilben (5 %ig) |
| 1,5 | 4,4-Diaminostilben×2 HCl |
| 1,5 | Anthrachinon 2,6-disulfonsäure (Na-salz) |
| 5 | 2,6-Dihydroxyanthrachinon |
| Vergleichsversuche nach US—A—3,552,928 | |
| 4—5 | Cysteinhydrochlorid×$H_2O$ |
| 5 | Tyrosin-Na-salz |
| 3 | L-Prolin-Na-salz |
| 3 | Alanin-Na-salz |
| 3—4 | L-Asparagin-Na-salz |
| 3—4 | L-Arginin-Na-salz |
| 4—5 | Tryptophan-Na-salz |
| Vergleichsversuche nach US—A—3,552,925 | |
| 3—4 | Zn-sulfat, Hämolyse |
| 0,5 | K-chlorid, starke Hämolyse |
| 2,5 | Na-acetat, starke Hämolyse |

1) WZ BASF
2) WZ Bayer
3) WZ Geigy
4) WZ Ciba
5) WZ Hoechst
6) WZ Cassella

Legende zu den Figuren 1—7

| | |
|---|---|
| 1 | Trägerfolie |
| 2 | Substratträger |
| 3 | Erythrozytenrückhaltezone |
| 4 | Schutznetz |
| 5 | Transportvlies (saugfähige Matrix) |
| 6 | Reagenzzone |
| 6a | Reagenzfilm |
| 6b | Gewebeschicht |
| 7 | Abdeckfolie |
| 8 | Reagenzträger |
| 9 | Indikatorpapier |
| 10 | Enzympapier |
| 11 | Klebestelle |

**Patentansprüche**

1. Verfahren zum Abtrennen von Erythrozyten aus Blut, dadurch gekennzeichnet, daß man das Blut mit einer Substanz aus der Gruppe der Verbindungen der allgemeinen Formel

$$R_1—Sp—R_2 \qquad (I)$$

in der

$R_1$ und $R_2$ gleich oder verschieden sind und $—SO_3H$, $—CO_2H$, $—NH_2$ oder $—OH$, insbesondere in Form ihrer Ionen,

$R_2$ auch eine Nitro-, Sulfonamid-, Carbonsäureamid-Gruppe oder Halogen- oder Alkoxygruppe und

Sp ein aromatisches oder heteroaromatisches Ringsystem aus mindestens zwei Ringen, die entweder anelliert oder direkt oder über —CH=CH—, —N=N—, —$CR_3$=N—, —$NR_3$—CO—$NR_4$—, —$CR_3R_4$— verbunden sind, wobei $R_3$ und $R_4$ Wasserstoff oder eine Alkylgruppe bedeuten und die Ringsysteme gegebenenfalls auch noch weitere Substituenten tragen können oder

wenn $R_1$ und $R_2$ zugleich —$NH_2$ bedeuten, auch eine Alkylengruppe mit 2 bis 10 C-Atomen darstellt, und

Primazingelb GRL®, Levafixbrilliantgelb E3G®, Levafix E-R2®, Levafixgoldgelb EG®, Blankophor REU®, Blankophor RA®, Blankophor BE®, Blankophor BA 267®, Blankophor BBH®, Blankophor RPA®, Blankophor BBU®, Tinopal BV® (C.I. 40630), Tinopal UP®, Uvitex NB®, Uvitex RT®, Uvitex CF®, Anthrachinondisulfonsäure-2,6 (Na-Salz), 2-Ethoxy-6,9-diaminoacridin, Primazin-Brilliantgelb 3-GL®, Remazolgelb FG®, Remazolgelb G®, Remazolgelb GNL®, Remazolgelb FGH®, Remazolbrilliantgelb GL®, Solidazolbrilliantgelb 4G®, Säuregelb 38® (C.I. 25135), Säuregelb 40® (C.I. 18950), Säuregelb 65® (C.I. 14170), Säuregelb 76® (C.I. 18850), Säuregelb 99® (C.I. 13900), Direktgelb 8® (C.I. 13920), Direktgelb 27® (C.I. 13950), Direktgelb 62® (C.I. 36900), Basic Yellow 11® (C.I. 48055), Thiazolgelb® (C.I. 19540) und Papiergelb RF® (C.I. 40000) in Kontakt bringt, gleichzeitig oder anschließend durch eine Filterschicht aus Glasfasern, Zellulose, Kunststoff oder Asbestfasern filtriert und das abgetrennte Plasma gewinnt.

2. Verfahren zum Abtrennen von Erythrozyten aus Blut gemäß Anspruch 1, dadurch gekennzeichnet, daß man das Blut mit einer Verbindung aus der Gruppe der Säurefarbstoffe, basischen Farbstoffe, Direktfarbstoffe, Beizenfarbstoffe, Reaktivfarbstoffe und optischen Aufheller in Kontakt bringt.

3. Verfahren nach einem der Ansprüche 1 oder 2, dadurch gekennzeichnet, daß die Verbindungen zum Abtrennen von Erythrozyten aus Blut in einer der Filterschicht vorgeschalteten Schicht enthalten sind oder dem Blut vorher zugefügt werden.

4. Verfahren nach einem der Ansprüche 1 oder 2, dadurch gekennzeichnet, daß die Verbindungen zum Abtrennen von Erythrozyten aus Blut in der Filterschicht enthalten sind.

5. Verfahren nach Anspruch 1 oder 2, dadurch gekennzeichnet, daß die Verbindungen zum Abtrennen von Erythrozyten aus Blut an die Matrix der Filterschicht oder einer vorgeschalteten Schicht gebunden sind.

6. Verwendung von Substanzen aus der Gruppe der Verbindungen der allgemeinen Formel

$$R_1—Sp—R_2 \qquad (I)$$

in der

$R_1$ und $R_2$ gleich oder verschieden sind und —$SO_3H$, —$CO_2H$, —$NH_2$ oder —OH, insbesondere in Form ihrer Ionen,

$R_2$ auch eine Nitro-, Sulfonamid-, Carbonsäureamid-Gruppe oder Halogen- oder Alkoxygruppe und

Sp ein aromatisches oder heteroaromatisches Ringsystem aus mindestens zwei Ringen, die entweder anelliert oder direkt oder über —CH=CH—, —N=N—, —$CR_3$=N—, —$NR_3$—CO—$NR_4$—, —$CR_3R_4$— verbunden sind, wobei $R_3$ und $R_4$ Wasserstoff oder eine Alkylgruppe bedeuten und die Ringsysteme gegebenenfalls auch noch weitere Substituenten tragen können oder wenn $R_1$ und $R_2$ zugleich —$NH_2$ bedeuten, auch eine Alkylengruppe mit 2 bis 10 C-Atomen darstellt, und

Primazingelb GRL®, Levafixbrilliantgelb E3G®, Levafix E-R2®, Levafixgoldgelb EG®, Blankophor REU®, Blankophor RA®, Blankophor BE®, Blankophor BA 267®, Blankophor BBH®, Blankophor RPA®, Blankophor BBU®, Tinopal BV® (C.I. 40630), Tinopal UP®, Uvitex NB®, Uvitex RT®, Uvitex CF®, Anthrachinondisulfonsäure-2,6 (Na-Salz), 2-Ethoxy-6,9-diaminoacridin, Primazin-Brilliantgelb 3-GL®, Remazolgelb FG®, Remazolgelb G®, Remazolgelb GNL®, Remazolgelb FGH®, Remazolbrilliantgelb GL®, Solidazolbrilliantgelb 4G®, Säuregelb 38® (C.I. 25135), Säuregelb 40® (C.I. 18950), Säuregelb 65® (C.I. 14170), Säuregelb 76® (C.I. 18850), Säuregelb 99® (C.I. 13900), Direktgelb 8® (C.I. 13920), Direktgelb 27® (C.I. 13950), Direktgelb 62® (C.I. 36900), Basic Yellow 11® (C.I. 48055), Thiazolgelb® (C.I. 19540) und Papiergelb RF® (C.I. 40000) zum Abtrennen von Erythrozyten aus Blut.

7. Verwendung von Verbindungen aus der Gruppe der Säurefarbstoffe, basischen Farbstoffe, Direktfarbstoffe, Beizenfarbstoffe, Reaktivfarbstoffe und optischen Aufheller zum Abtrennen von Erythrozyten aus Blut.

8. Verwendung von Verbindungen gemäß Anspruch 6, wobei

Sp ein aromatisches oder heteroaromatisches Ringsystem darstellt, in dem die Substituenten $R_1$ und $R_2$ an verschiedenen Ringen positioniert sind.

9. Verwendung von Verbindungen gemäß Anspruch 6 oder 8, wobei die Ringsysteme weitere Substituenten enthalten, die —$SO_3H$, —$CO_2H$, —$NH_2$, Alkylamin, Dialkylamin, —OH, —$NO_2$, Halogen, Alkoxy, Alkyl oder —$CONH_2$ bedeuten.

10. Verwendung von Verbindungen gemäß Anspruch 6, wobei

$R_1$ und $R_2$ eine $NH_2$-Gruppe, insbesondere in Form ihrer Ionen und

Sp eine Alkylengruppe mit 2—6 C-Atomen bedeuten.

11. Mittel zum Abtrennen von Erythrozyten aus Blut, dadurch gekennzeichnet, daß es Substanzen aus der Gruppe der Verbindungen der allgemeinen Formel

$$R_1—Sp—R_2 \qquad (I)$$

in der

$R_1$ und $R_2$ gleich oder verschieden sind und —$SO_3H$, —$CO_2H$, —$NH_2$ oder —OH, insbesondere in Form ihrer Ionen,

$R_2$ auch eine Nitro-, Sulfonamid-, Carbonsäureamid-Gruppe oder Halogen- oder Alkoxygruppe und

Sp ein aromatisches oder heteroaromatisches Ringsystem aus mindestens zwei Ringen, die entweder anelliert oder direkt oder über —CH=CH—, —N=N—, —$CR_3$=N—, —$NR_3$—CO—$NR_4$—, —$CR_3R_4$— verbunden sind, wobei $R_3$ und $R_4$ Wasserstoff oder eine Alkylgruppe bedeuten und die Ringsysteme gegebenenfalls auch noch weitere Substituenten tragen können oder wenn $R_1$ und $R_2$ zugleich —$NH_2$ bedeuten, auch eine Alkylengruppe mit 2 bis 10 C-Atomen darstellt, und

Primazingelb GRL®, Levafixbrilliantgelb E3G®, Levafix E-R2®, Levafixgoldgelb EG®, Blankophor REU®, Blankophor RA®, Blankophor BE®, Blankophor BA 267®, Blankophor BBH®, Blankophor RPA®, Blankophor BBU®, Tinopal BV® (C.I. 40630), Tinopal UP®, Uvitex NB®, Uvitex RT®, Uvitex CF®, Anthrachinondisulfonsäure-2,6 (Na-Salz), 2-Ethoxy-6,9-diaminoacridin, Primazin-Brilliantgelb 3-GL®, Remazolgelb FG®, Remazolgelb G®, Remazolgelb GNL®, Remazolgelb FGH®, Remazolbrilliantgelb GL®, Solidazolbrilliantgelb 4G®, Säuregelb 38® (C.I. 25135), Säuregelb 40® (C.I. 18950), Säuregelb 65® (C.I. 14170), Säuregelb 76® (C.I. 18850), Säuregelb 99® (C.I. 13900), Direktgelb 8® (C.I. 13920), Direktgelb 27® (C.I. 13950), Direktgelb 62® (C.I. 36900), Basic Yellow 11® (C.I. 48055), Thiazolgelb® (C.I. 19540) und Papiergelb RF® (C.I. 40000) in trägergebundener Form enthält.

12. Mittel zur Abtrennung von Erythrozyten aus Blut gemäß Anspruch 11, dadurch gekennzeichnet, daß es Verbindungen aus der Gruppe der Säurefarbstoffe, basischen Farbstoffe, Direktfarbstoffe, Beizenfarbstoffe, Reaktivfarbstoffe und optischen Aufheller enthält.

13. Mittel gemäß einem der Ansprüche 11 oder 12, dadurch gekennzeichnet, daß der Träger aus einem saugfähigen oder nicht saugfähigen durchlässigen Material besteht.

14. Mittel gemäß einem der Ansprüche 11 oder 12, dadurch gekennzeichnet, daß die trägergebundenen Verbindungen einer Filterzone vorgeschaltet sind.

**Revendications**

1. Procédé pour isoler des érythrocytes à partir du sang, caractérisé en ce que l'on met en contact le sang avec une substance du groupe des composés de formule générale

$$R_1—Sp—R_2 \qquad (I)$$

où

$R_1$ et $R_2$ sont identiques ou différents et représentent un groupe —$SO_3H$, —$CO_2H$, —$NH_2$ ou —OH, en particulier sous la forme de leurs ions.

$R_2$ représente également un groupe nitro, sulfonamido, carboxyamido ou un atome d'halogène ou un groupe alcoxy, et

Sp représente un système cyclique aromatique ou hétéroaromatique constitué d'au moins deux cycles qui sont soit annelles, soit relies directement ou par l'intermédiaire d'un groupe —CH=CH—, —N=N—, —$CR_3$=N—, —$NR_3$—CO—$NR_4$—, —$CR_3R_4$—, $R_3$ et $R_4$ représentant un atome d'hydrogène ou un groupe alkyle et le système cyclique pouvant porter éventuellement encore d'autres substituants ou lorsque $R_1$ et $R_2$ représentent sous deux —$NH_2$, il représenté également un groupe alkylène comportant 2 à 10 atomes de C, et Primazingelb GRL®, Lavafixbrilliantgelb E3G®, Levafix E-R2®, Levafixgoldgelb EG®, Blankophor REU®, Blankophor RA®, Blankophor BE®, Blankophor BA 267®, Blankophor BBH®, Blankophor RPA®, Blankophor BBU®, Tinopal BV® (C.I. 40630), Tinopal UP®, Uvitex NB®, Uvitex RT®, Uvitex CF®, acide anthraquinone-2,6-disulfonique (sel de Na), 2-ethoxy-6,9-diaminoacridine, Primazin-Brilliantgelb 3-GL®, Remazolgelb FG®, Remazolgelb G®, Remazolgelb GNL®, Remazolgelb FGH®, Remazolbrilliantgelb GL®, Solidazolbrilliantgelb 4G®, Säuregelb 38® (C.I. 25135), Sauregelb 40® (C.I. 18950), Sauregelb 65® (C.I. 14170), Säuregelb 76® (C.I. 18850), Säuregelb 99® (C.I. 13900), Direktgelb 8® (C.I. 13920), Direktgelb 27® (C.I. 13950), Direktgelb 62® (C.I. 36900), Basic Yellow 11® (C.I. 48055), Thiazolgelb® (C.I. 19540) et Papiergelb RF® (C.I. 40000), et simultanément ou subséquemment, on filtre le sang à travers une couche de filtration constituée de fibres de verre, de cellulose, de matières synthétiques ou de fibres d'amiante et on recupère le plasma séparé.

2. Procédé pour isoler des érythrocytes à partir du sang, selon la revendication 1, caractérisé en ce que l'on met le sang en contact avec un compose du groupe constitué par les colorants acides, les colorants basiques, les colorants directs, les colorants sur mordant, les colorants réactifs et les azurants optiques.

3. Procédé selon l'une des revendications 1 ou 2, caractérisé en ce que les composés destinés à isoler les érythrocytes à partir du sang sont contenus dans une couche montée avant la couche de filtration ou sont ajoutés prealablement au sang.

4. Procédé selon l'une des revendications 1 ou 2, caractérisé en ce que les composés destinés à isoler les érythrocytes à partir du sang sont contenus dans la couche de filtration.

5. Procédé selon l'une des revendications 1 ou 2, caractérisé en ce que les composés destinés à isoler les érythrocytes à partir du sang sont liés à la matrice de la couche de filtration ou d'une couche montée devant la précédente.

6. Utilisation de substances du groupe des composes de formule générale

$$R_1—Sp—R_2 \quad (I)$$

où

$R_1$ et $R_2$ sont identiques ou differents et représentent un groupe $—SO_3H$, $—CO_2H$, $—NH_2$ ou —OH, en particulier sous la forme de leurs ions,

$R_2$ représente également un groupe nitro, sulfonamido, carboxyamido ou un atome d'halogène ou un groupe alcoxy, et

Sp représente un système cyclique aromatique ou héteroaromatique constitué d'au moins deux cycles qui sont soit annellés, soit reliés directement ou par l'intermédiaire d'un groupe —CH=CH—, —N=N—, $—CR_3=N—$, $—NR_3—CO—NR_4—$, $—CR_3R_4—$, $R_3$ et $R_4$ représentant un atome d'hydrogène ou un groupe alkyle et le système cyclique pouvant porter eventuellement encore d'autres substituants ou lorsque $R_1$ et $R_2$ représentent tous deux $—NH_2$, il représente également un groupe alkylène comportant 2 à 10 atomes de C, et Primazingelb GRL®, Lavafixbrilliantgelb E3G®, Levafix E-R2®, Levafixgoldgelb EG®, Blankophor REU®, Blankophor RA®, Blankophor BE®, Blankophor BA 267®, Blankophor BBH®, Blankophor RPA®, Blankophor BBU®, Tinopal BV® (C.I. 40630), Tinopal UP®, Uvitex NB®, Uvitex RT®, Uvitex CF®, acide anthraquinone-2,6-disulfonique (sel de Na), 2-éthoxy-6,9-diaminoacridine, Primazin-Brilliantgelb 3-GL®, Remazolgelb FG®, Remazolgelb G®, Remazolgelb GNL®, Remazolgelb FGH®, Remazolbrilliantgelb GL®, Solidazolbrilliantgelb 4G®, Säuregelb 38® (C.I. 25135), Säuregelb 40≤ (C.I. 18950), Säuregelb 65® (C.I. 14170), Säuregelb 76® (C.I. 18850), Säuregelb 99® (C.I. 13900), Direktgelb 8® (C.I. 13920), Direktgelb 27® (C.I. 13950), Direktgelb 62® (C.I. 36900), Basic Yellow 11® (C.I. 48055), Thiazolgelb® (C.I. 19540) et Papiergelb RF® (C.I. 40000), pour isoler des érythrocytes à partir du sang.

7. Utilisation de composes du groupe constitué par les colorants acides, les colorants basiques, les colorants directs, les colorants sur mordant, les colorants réactifs et les azurants optiques, pour isoler des érythrocytes à partir du sang.

8. Utilisation des composes selon la revendication 6, où Sp représenté un système cyclique aromatique ou hétéroaromatique, dans lequel les substituants $R_1$ et $R_2$ sont placés sur des cycles différents.

9. Utilisation de composés selon la revendication 6 ou 8, où les systèmes cycliques contiennent d'autres substituants, tels que les groupes $—SO_3H$, $—CO_2H$, $—NH_2$, alkylamine, dialkylamine, —OH, $—NO_2$, halogène, alcoxy, alkyle ou $—CONH_2$.

10. Utilisation des composés selon la revendication 6, où $R_1$ et $R_2$ representent un groupe $—NH_2$, en particulier sous la forme de ses ions, et Sp représente un groupe alkylène, comportant 2—6 atomes de C.

11. Agent pour isoler des érythrocytes à partir du sang, caractérisé en ce qu'il contient des substances du groupe des composés de formule générale

$$R_1—Sp—R_2 \quad (I)$$

où

$R_1$ et $R_2$ sont identiques ou différents et représentent un groupe $—SO_3H$, $—CO_2H$, $—NH_2$ ou —OH, en particulier sous la forme de leurs ions,

$R_2$ représente également un groupe nitro, sulfonamido, carboxyamido ou un atome d'halogène ou un groupe alcoxy, et

Sp représente un système cyclique aromatique ou hétéroaromatique constitué d'au moins deux cycles qui sont annelles, soit reliès directement ou par l'intermédiaire d'un groupe —CH=CH—, —N=N—, $—CR_3=N—$, $—NR_3—CO—NR_4—$, $—CR_3R_4—$, $R_3$ et $R_4$ représentant un atome d'hydrogène ou un groupe alkyle et le système cyclique pouvant porter éventuellement encore d'autres substituants ou lorsque $R_1$ et $R_2$ représentent tous deux $—NH_2$, il représente également un groupe alkylène comportant 2 à 10 atomes de C, et Primazingelb GRL®, Lavafixbrilliantgelb E3G®, Levafix E-R2® Levafixgoldgelb EG®, Blankophor REU®, Blankophor RA®, Blankophor BE®, Blankophor BA 267®, Blankophor BBH®, Blankophor RPA®, Blankophor BBU®, Tinopal BV® (C.I. 40630), Tinopal UP®, Uvitex NB®, Uvitex RT®, Uvitex CF®, acide anthraquinone-2,6-disulfonique (sel de Na), 2-éthoxy-6,9-diaminoacridine, Primazin-Brilliantgelb 3-GL®, Remazolgelb FG®, Remazolgelb G®, Remazolgelb GNL®, Remazolgelb FGH®, Remazolbrilliantgelb GL®, Solidazolbrilliantgelb 4G®, Säuregelb 38® (C.I. 25135), Säuregelb 40® (C.I. 18950), Säuregelb 65® (C.I. 14170), Säuregelb 76® (C.I. 18850), Säuregelb 99® (C.I. 13900), Direktgelb 8® (C.I. 13920), Direktgelb 27® (C.I. 13950), Direktgelb 62® (C.I. 36900), Basic Yellow 11® (C.I. 48055), Thiazolgelb® (C.I. 19540) et Papiergelb RF® (C.I. 40000), sous une forme liée à un support.

12. Agent destine à isoler des érythrocytes à partir du sang, selon la revendication 11, caractérisé en ce qu'il contient des composés du groupe constitué par les colorants acides, les colorants basiques, les colorants directs, les colorants sur mordant, les colorants réactifs et les azurants optiques.

13. Agent selon l'une des revendications 11 et 12, caractérisé en ce que le support est constitué par un matériau perméable, absorbant ou non absorbant.

14. Agent selon l'une des revendications 11 et 12, caractérisé en ce que les composés liés à un support sont placés avant une zone de filtration.

**Claims**

1. Process for the separation of erythrocytes from blood, characterised in that one brings the blood into contact with a substance of the group of compounds of the general formula

$$R_1\text{—Sp—}R_2 \qquad (I)$$

in which $R_1$ and $R_2$ are the same or different and represent —$SO_3H$, —$CO_2H$, —$NH_2$ or —OH, especially in the form of their ions, $R_2$ also a nitro, sulphonamide or carboxylic acid amide group or halogen or alkoxy group and Sp an aromatic or heteroaromatic ring system of at least two rings which are either annellated or bound directly or via —CH=CH—, —N=N—, —$CR_3$=N—, —$NR_3$—CO—$NR_4$—, —$CR_3R_4$—, whereby $R_3$ and $R_4$ signify hydrogen or an alkyl group and the ring systems can possibly also carry further substituents or, when $R_1$ and $R_2$ simultaneously signify —$NH_2$, also represents an alkylene group with 2 to 10 C-atoms and Primazin Yellow GRL®, Levafix Brilliant Yellow E3G®, Levafix E-R2®, Levafix Gold Yellow EG®, Blancophor REU®, Blancophor RA®, Blancophor BE®, Blancophor BA 267®, Blancophor BBH®, Blancophor RPA®, Blancophor BBU®, Tinopal BV® (C.I. 40630), Tinopal UP®, Uvitex NB®, Uvitex RT®, Uvitex CF®, anthraquinone-2,6-disulphonic acid (Na salt), 2-ethoxy-6,9-diaminoacridine, Primazin Brilliant Yellow 3-GL®, Remazol Yellow FG®, Remazol Yellow G®, Remazol Yellow GNL®, Remazol FGH®, Remazol Brilliant Yellow GL®, Solidazol Brilliant Yellow 4G®, Acid Yellow 38® (C.I. 25135), Acid Yellow 40® (C.I. 18950), Acid Yellow 65® (C.I. 14170), Acid Yellow 76® (C.I. 18850), Acid Yellow 99® (C.I. 13900), Direct Yellow 8® (C.I. 13920), Direct Yellow 27® (C.I. 13950), Direct Yellow 62® (C.I. 36900), Basic Yellow 11® (C.I. 48055), Thiazole Yellow® (C.I. 19540) and Paper Yellow RF® (C.I. 40000), filters simultaneously or subsequently through a filter layer of glass fibres, cellulose, synthetic resin or asbestos fibres and recovers the separated plasma.

2. Process for the separation of erythrocytes from blood according to claim 1, characterised in that the blood is brought into contact with a compound of the group of the acid dyestuffs, basic dyestuffs, direct dyestuffs, mordant dyestuffs, reactive dyestuffs and optical brightening agents.

3. Process according to one of claims 1 or 2, characterised in that the compounds for the separation of erythrocytes from blood are contained in a layer placed before the filter layer or are previously added to the blood.

4. Process according to one of claims 1 or 2, characterised in that the compounds for the separation of erythrocytes from the blood are contained in the filter layer.

5. Process according to claim 1 or 2, characterised in that the compounds for the separation of erythrocytes from blood are bound to the matrix of the filter layer or of a layer placed therebefore.

6. Use of substances from the group of compounds of the general formula

$$R_1\text{—Sp—}R_2 \qquad (I)$$

in which $R_1$ and $R_2$ are the same or different and signify —$SO_3H$, —$CO_2H$, —$NH_2$ or —OH, especially in the form of their ions, $R_2$ also a nitro, sulphonamide, carboxylic acid amide group or halogen or alkoxy group and Sp an aromatic or heteroaromatic ring system of at least two rings which are either anellated or bound directly or via —CH=CH—, —N=N—, —$CR_3$=N—, —$NR_3$—CO—$NR_4$—, —$CR_3R_4$—, whereby $R_3$ and $R_4$ signify hydrogen or an alkyl group and the ring systems can possibly also carry further substituents or, when $R_1$ and $R_2$ simultaneously signify —$NH_2$, also represents an alkylene group with 2 to 10 C-atoms, and Primazin Yellow GRL®, Levafix Brilliant Yellow E3G®, Levafix E-R2®, Levafix Gold Yellow EG®, Blancophor REU®, Blancophor RA®, Blancophor BE®, Blancophor BA 267®, Blancophor BBH®, Blancophor RPA®, Blancophor BBU®, Tinopal BV® (C.I. 40630), Tinopal UP®, Uvitex NB®, Uvitex RT®, Uvitex CF®, anthraquinone-2,6-disulphonic acid (Na salt), 2-ethoxy-6,9-diaminoacridine, Primazin Brilliant Yellow 3-GL®, Remazol Yellow FG®, Remazol Yellow G®, Remazol Yellow GNL®, Remazol Yellow FGH®, Remazol Brilliant Yellow GL®, Solidazol Brilliant Yellow 4G®, Acid Yellow 38® (C.I. 25135), Acid Yellow 40® (C.I. 18950), Acid Yellow 65® (C.I. 14170), Acid Yellow 76® (C.I. 18850), Acid Yellow 99® (C.I. 13900), Direct Yellow 8® (C.I. 13920), Direct Yellow 27® (C.I. 13950), Direct Yellow 62® (C.I. 36900), Basic Yellow 11® (C.I. 48055), Thiazole Yellow® (C.I. 19540) and Paper Yellow RF® (C.I. 40000) for the separation of erythrocytes from blood.

7. Use of compounds of the group of the acid dyestuffs, basic dyestuffs, direct dyestuffs, mordant dyestuffs, reactive dyestuffs and optical brightening agents for the separation of erythrocytes from blood.

8. Use of compounds according to claim 6, whereby Sp represents an aromatic or heteroaromatic ring system in which the substituents $R_1$ and $R_2$ are positioned on different rings.

9. Use of compounds according to claim 6 or 8, whereby the ring systems contain further substituents which signify —$SO_3H$, —$CO_2H$, —$NH_2$, alkylamine, dialkylamine, —OH, —$NO_2$, halogen, alkoxy, alkyl or —$CONH_2$.

10. Use of compounds according to claim 6, whereby $R_1$ and $R_2$ signify an $NH_2$ group, especially in the form of its ions, and Sp and alkylene group with 2—6 C-atoms.

11. Agent for the separation of erythrocytes from blood, characterised in that it contains substances of the group of the compounds of the general formula

$R_1—Sp—R_2$ (I)

in which $R_1$ and $R_2$ are the same or different and signify $—SO_3H$, $—CO_2H$, $—NH_2$ or $—OH$, especially in the form of their ions, $R_2$ also a nitro, sulphonamide, carboxylic acid amide group or halogen or alkoxy group and Sp an aromatic or heteroaromatic ring system of at least two rings which are either anellated or bound directly or via $—CH=CH—$, $—N=N—$, $—CR_3=N—$, $—NR_3—CO—NR_4—$, $—CR_3R_4—$, whereby $R_3$ and $R_4$ signify hydrogen or an alkyl group and the ring systems can possibly also contain further substituents or when $R_1$ and $R_2$ simultaneously signify $—NH_2$, also represent an alkylene group with 2 to 10 C-atoms and Primazin Yellow GRL®, Levafix Brilliant Yellow E3G®, Levafix E-R2®, Levafix Gold Yellow EG®, Blancophor REU®, Blancophor RA®, Blancophor BE®, Blancophor BA 267®, Blancophor BBH®, Blancophor RPA®, Blancophor BBU®, Tinopal BV® (C.I. 40630), Tinopal UP®, Uvitex NB®, Uvitex RT®, Uvitex CF®, anthraquinone-2,6-disulphonic acid (Na salt), 2-ethoxy-6,9-diaminoacridine, Primazin Brilliant Yellow 3-GL®, Remazol Yellow FG®, Remazol Yellow G®, Remazol Yellow GNL®, Remazol Yellow FGH®, Remazol Brilliant Yellow GL®, Solidazol Brilliant Yellow 4G®, Acid Yellow 38® (C.I. 25135), Acid Yellow 40® (C.I. 18950), Acid Yellow 65® (C.I. 14170), Acid Yellow 76® (C.I. 18850), Acid Yellow 99® (C.I. 13900), Direct Yellow 8® (C.I. 13920), Direct Yellow 27® (C.I. 13950), Direct Yellow 62® (C.I. 36900), Basic Yellow 11® (C.I. 48055), Thiazole Yellow® (C.I. 19540) and Paper Yellow RF® (C.I. 40000) in carrier-bound form.

12. Agent for the separation of erythrocytes from blood according to claim 11, characterised in that it contains compounds of the group of the acid dyestuffs, basic dyestuffs, direct dyestuffs, mordant dyestuffs, reactive dyestuffs and optical brightening agents.

13. Agent according to one of claims 11 or 12, characterised in that the carrier consists of an absorbent or non-absorbent permeable material.

14. Agent according to one of claims 11 or 12, characterised in that the carrier-bound compounds are placed before a filter zone.

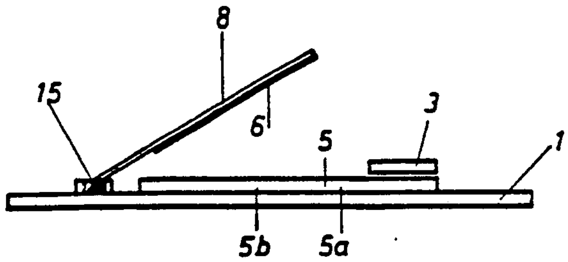


Fig.1

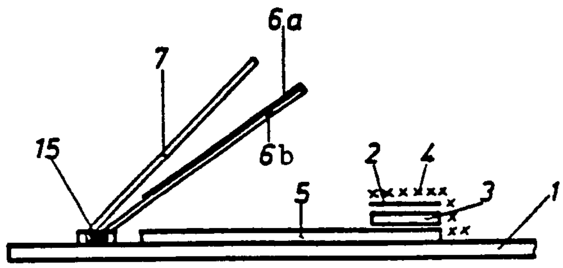


Fig.2

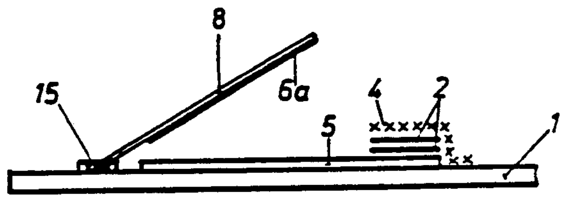


Fig.3

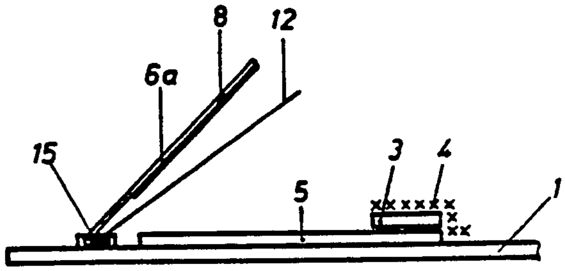


Fig.4

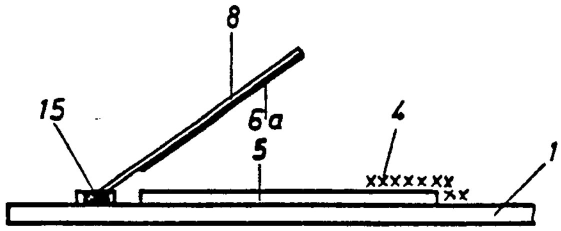


Fig. 5

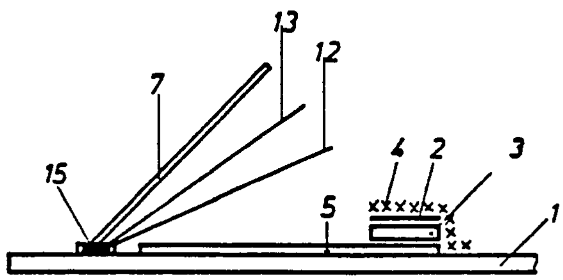


Fig.6

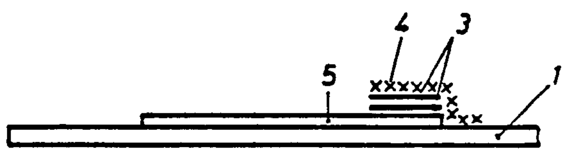


Fig.7